# EUROPÄISCHE PATENTSCHRIFT

(11) **EP 2 170 804 B1**
(45) Veröffentlichungstag und Bekanntmachung des Hinweises auf die Patenterteilung: **28.08.2013**
(21) Anmeldenummer: 08760854.3
(22) Anmeldetag: 11.06.2008
(51) Int. Cl.: C07C 205/37, C07C 201/12

(54) **Verfahren zur Herstellung von 2-Phenoxyacetalen und den daraus korrespondierenden 2-Phenoxycarbaldehyden**
Method of producing 2-phenoxyacetals and the corresponding 2-phenoxy-carbaldehydes therefrom
Procédé de fabrication de 2-phénoxyacétals et des 2-phénoxycarbaldéhydes correspondants

(30) Priorität: 22.06.2007 DE 102007028925; 31.10.2007 DE 102007052313
(43) Veröffentlichungstag der Anmeldung: 07.04.2010
(73) Patentinhaber: Saltigo GmbH, 40764 Langenfeld (DE)
(72) Erfinder: MÜLLER, Thomas-Norbert, 40789 Monheim (DE); DOCKNER, Michael, 50674 Köln (DE)
(74) Vertreter: Pettrich, Klaus-Günter
(86) Internationale Anmeldenummer: PCT/EP2008/057303
(87) Internationale Veröffentlichungsnummer: WO 2009/000651

(56) Entgegenhaltungen:
- EP-A- 0 953 565
- EP-A- 1 103 551
- EP-A- 1 698 628
- WO-A-00/40554
- WO-A-97/43272
- WO-A-03/033476
- WO-A-2004/063192
- WO-A-2004/069829
- WO-A-2004/094386
- WO-A-2006/066174
- WO-A-2008/062740
- US-A- 2 553 555
- US-A- 2 629 741
- SMITH R L ET AL: "11,12-SECOPROSTAGLANDINS. 3. 8-ALKYLTHIO(SULFINYL AND SULFONYL)-12-HYDROXYALKANOIC ACIDS AND RELATED COMPOUNDS" JOURNAL OF MEDICINAL CHEMISTRY, US AMERICAN CHEMICAL SOCIETY. WASHINGTON, Bd. 20, Nr. 4, 1. Januar 1977 (1977-01-01), Seiten 540-547, XP009009170 ISSN: 0022-2623
- KURT PILGRAM AND GLENN E. POLLARD: "1,3-Oxazilidin-4-ones. Synthesis and Configuratin of cis and trans isomers." JOURNAL OF HETEROCYCLIC CHEMISTRY, Bd. 14, 1977, Seiten 1029-1033, XP002493541
- CHIOCCARA F ET AL: "NEW BENZOXAZINE AND BENZOTHIAZINE DYES" TETRAHEDRON, ELSEVIER SCIENCE PUBLISHERS, AMSTERDAM, NL, Bd. 32, Nr. 12, 1. Januar 1976 (1976-01-01), Seiten 1407-1409, XP009001144 ISSN: 0040-4020
- ANSAR M ET AL: "3-Benzo[b]furyl- and 3-benzo[b]thienylaminobutyric acids as GABAB ligands. Synthesis and structure-activity relationship studies" EUROPEAN JOURNAL OF MEDICINAL CHEMISTRY, EDITIONS SCIENTIFIQUE ELSEVIER, PARIS, FR, Bd. 31, Nr. 6, 1. Januar 1996 (1996-01-01), Seiten 449-460, XP004040069 ISSN: 0223-5234

## Beschreibung

Die Erfindung betrifft ein neues Verfahren zur Herstellung von 2-Phenoxyacetalen und den dazu korrespondierenden 2-Phenoxycarbaldehyden. Diese Verbindungen sind wichtige Zwischenprodukte für die Herstellung pharmazeutischer Wirkstoffe.

Der bislang einzige beschriebene Weg zu 2-Phenoxyacetalen beinhaltet als Schlüsselschritt eine Rosenmundreaktion zur Darstellung des Aldehyds (Abb. 1). Das dafür benötigte Säurechlorid ist durch Reaktion eines α-Halogencarbonsäureesters mit dem entsprechenden Phenolat, gefolgt von einer Verseifung des gebildeten 2-Phenoxyesters und anschließender Behandlung mit Thionylchlorid, zugänglich. Das entsprechende Acetal wird durch Behandlung des Aldehyds mit Methanol in Gegenwart katalytischer Mengen Säure dargestellt. Auf diesem Wege wurden in erster Linie aromatische Verbindungen mit elektronreichen Substituenten (R = OMe, Alkyl) dargestellt [Kwiecien, Polish J. Chem. 2004, 78, 249 - 254; Synth. Commun. 2005, 35, 2223 - 2250]. Ein Nachteil dieser Methode ist der sehr langwierige 5-stufige Syntheseweg.

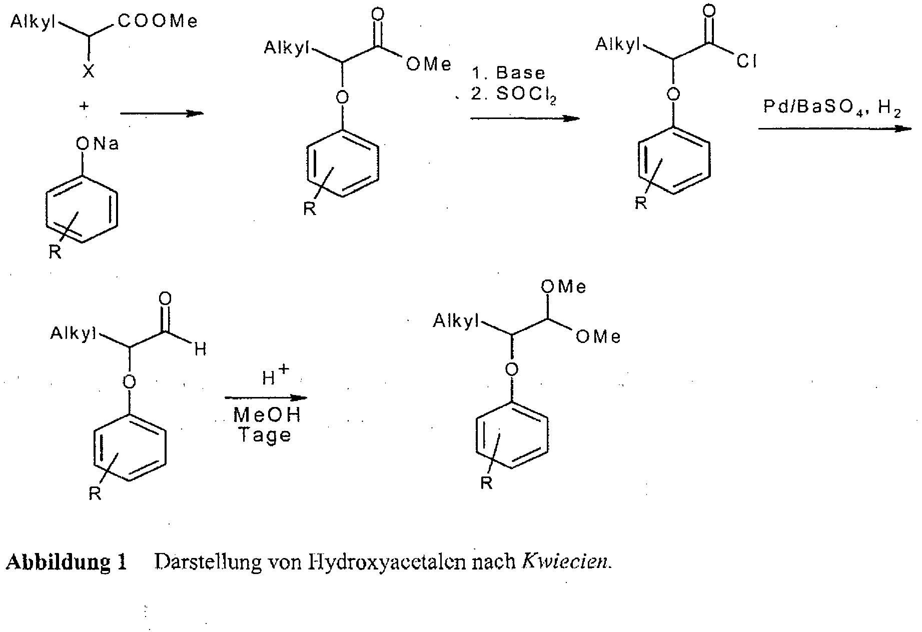

Die Synthese von 2-Phenoxycarbonylverbindungen mit elektronenarmen Substituenten aus den entsprechenden Phenolaten und α-halogenierten Carbonylverbindungen gelingt unter wesentlich drasüscheren Bedingungen. So sind zur Umsetzung hohe Temperaturen von 190 - 200 °C [Bischorf, Chem. Ber. 1900, 33, 1603 - 1611] oder lange Reaktionszeiten [Harfenist, J. Org. Chem. 1971, 36, 1171 - 1175] notwendig. Eine Variante zur Verätherung beinhaltet die Deprotonierung des Phenols mit Natriumhydrid (Abb. 2). Nachteilig ist dabei die Selbstentzündlichkeit von Natriumhydrid und die damit einhergehenden notwendigen sicherheitstechnischen Maßnahmen. Um den gewünschten Carbaldehyd zu erhalten, muss zunächst die durch Verseifung des Esters erhaltene Carbonsäure zum Alkohol reduziert werden, worauf dann dieser zum Zielprodukt oxidiert wird [Yi, Bull. Korean Chem. Soc. 2004, 25, 1003 - 1008]. Ein weiterer Nachteil dieser Methode ist die lange Syntheseroute und die damit einhergehende geringe Ausbeute (32 % über 5 Stufen).

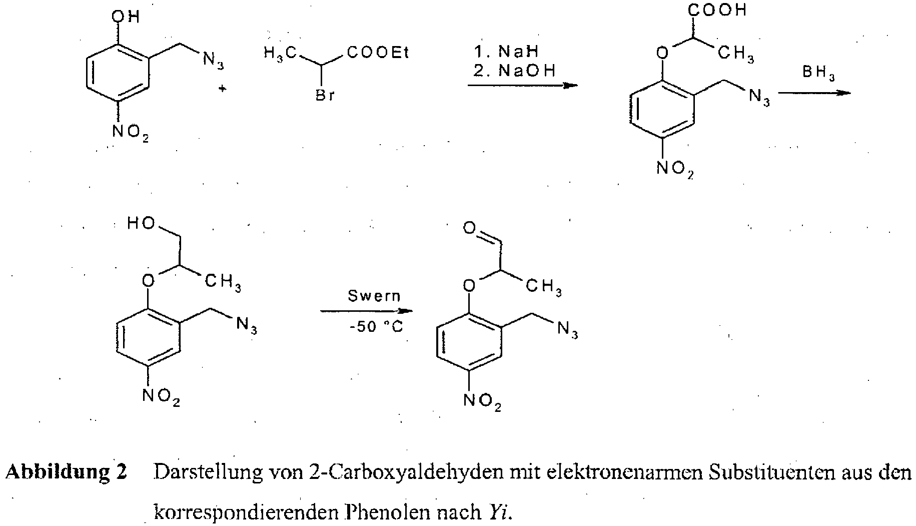

Die Darstellung von Arylalkylethem mit elektrodenarmen Substituenten gelingt auch durch Umsetzung von aliphatischen Alkoholen mit den entsprechenden Arylhalogeniden (Abb. 3).

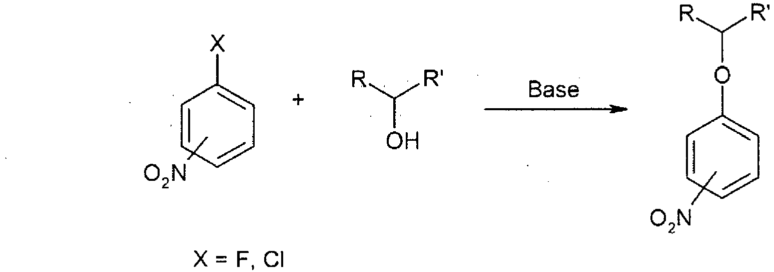

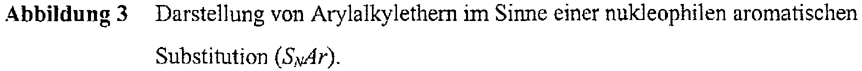

Typischerweise wird der Alkohol direkt als Alkalimetallsalz mit dem Arylfluorid oder -chlorid zur Reaktion gebracht, oder *in situ* durch Zugabe von Alkalimetallhydriden oder starke Basen wie Alkalimetallhexamethyldisilazane oder Alkalimetall-tert.-butanolaten erzeugt. Die Verwendung von Alkalimetallhydriden im großen Maßstab ist aufgrund ihrer extrem starken Reaktionsfähigkeit mit Wasser und ihrer schlechten Handhabbarkeit nicht wünschenswert und stellt ein großes Sicherheitsrisiko dar.

Eine weitere Möglichkeit ist die Verwendung eines zweiphasigen Systems, in dem Alkohol und Arylhalogenid in einem nicht mit Wasser mischbarem Lösungsmittel wie Toluol gelöst sind, und die zur Deprotonierung des Nucleophils benötigte Base in einer wässrigen Lösung vorliegt. Als Ermittler werden Phasentransferkatalysatoren, typischerweise quaternäre Ammoniumhalogenide eingesetzt.

Die Verwendung von Chloraromaten führt jedoch zu stark schwankenden Ausbeuten aufgrund unerwünschter Nebenreaktionen des aromatischen Einsatzmaterials. So werden bei der Wahl ungeeigneter Reaktionsbedingungen z.B. in großem Maße aus Chlornitrobenzolen die Nitrophenole als Nebenkomponente gebildet.

Aufgrund ihrer höheren Reaktivität können Arylfluoride mit primären, sekundären und tertiären Alkoholaten als Nucleophile zur Reaktion gebracht werden [Beispiel für die Reaktion mit tertiären Alkoholen: EP1564201A1]. Die Umsetzung von Arylchloriden mit tertiären Alkoholen gelingt laut Literatur entweder nur in sehr geringem Maße oder überhaupt nicht [Paradisi/Scorrano, J. Org. Chem. 1983, 48, 3022 - 3026; Seguchi, Yukagaku 1982, 31, 609 - 611.]. Für die Reaktion von Arylchloriden mit unfunktionalisierten sekundären Alkoholaten gibt es nur wenige Beispiele. Eine Umsetzung ist beispielhaft in Abb. 4 dargestellt [Bansho, JP 2002-255905]. Die Ausbeute betrug 86 %.

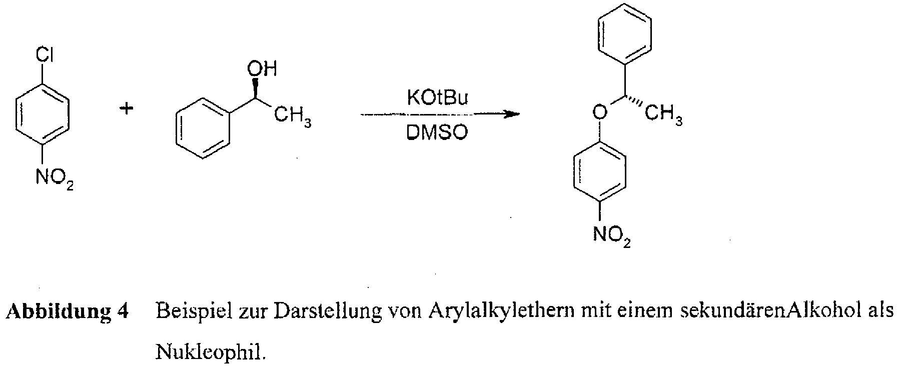

Für die Darstellung eines Arylalkylethers mit einer als Acetal geschützten Carbonylfunktionalität in der Seitenkette durch nukleophile Substitution (Abb. 5) findet sich nur ein Beispiel in der Literatur [Kawamatsu, Chem. Pharm. Bull. 1984, 32, 2267 - 2278]. Dabei wurde der unten dargestellte primäre Alkohol mit dem gegenüber dem Chlorderivat wesentlich reaktiveren 4-Fluomitrobenzol zur Reaktion gebracht. Das gewünschte Produkt wurde in 87 % Ausbeute erhalten. Problematisch ist hier wieder die Verwendung von Natriumhydrid als Base und die damit einhergehenden aufwändigen Maßnahmen zum sicheren Umgang mit dieser Substanz.

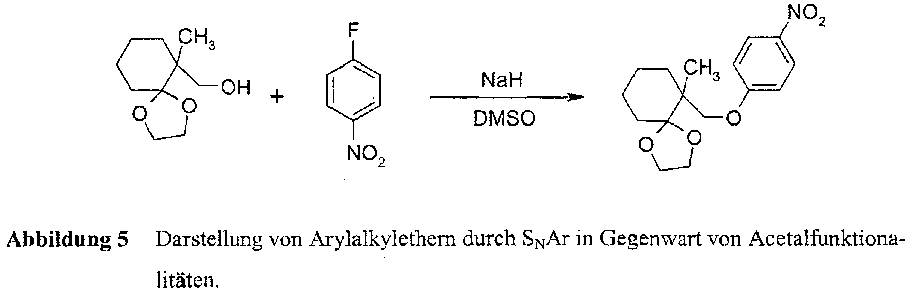

Aufgabe der vorliegenden Erfindung war es, ein neues wirtschaftlicheres Verfahren zur Herstellung von 2-Phenoxyacetalen und den daraus korrespondierenden 2-Phenoxycarbaldehyden mit verbesserter Ausbeute zur Verfügung zu stellen.

In der vorliegenden Erfindung gelingt es, den Syntheseweg zu den 2-Phenoxyacetalen von fünf bzw. sechs Schritten auf einen Schritt zu reduzieren (Abb. 6). Die dafür benötigen 2-Hydroxyacetale sind leicht in technischen Mengen verfügbar. Die Gesamiausbeute ausgehend vom Aldehyd konnte gegenüber der Methode von *Yi* (Abb. 5) von 31 % auf 55 % gesteigert werden. Weiterhin werden im technischen Maßstab problematische Syntheseoperationen wie eine Rosenmund-Reaktion oder eine Swern-Oxidation vermieden. Die Umsetzung von 4-Chlornitrobenzol mit sekundären Alkoholen ist bislang nur für nicht funktionalisierte Alkohole beschrieben.

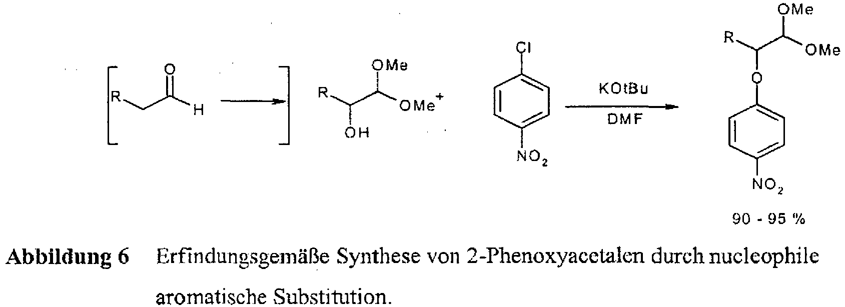

Weitere Verfahren zur Herstellung von 2-Phenoxyacetaldehyd-acetal-Verbindungen durch nucleophile aromatische Substituenten eines aktivierten aromatischen Halogenids mit 2-Hydroxy-acetaldehyd-acetalen in Gegenwart von Natriumhydrid in wasserfreiem DMF sind aus WO 03/033476A, WO2006/066174A und EP 1103551A bekannt.

Es wurde ein Verfahren gefunden, bei dem elekwonenarme Fluor- und Chloraromaten mit 2Hydroxyacetalen, 2-Phenoxyacetalen überraschenderweise unter milden Bedingungen umgesetzt werden können. Dabei wird das Alkoholderivat in Gegenwart einer starken Base deprotoniert und anschließend mit dem gewünschten Chloraromaten umgesetzt.

Gegenstand der Erfindung ist daher ein Verfahren zur Herstellung von 2-Phenoxyacetalen der allgemeinen Formel (Ia) 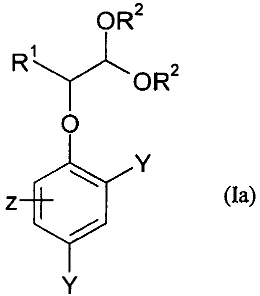 in der
- R¹: ein verzweigter oder unverzweigter C₁-C₂-Alkylrest, ein elektronenarmer, mit Halogen, einer -NO₂, -CN, -CF₃, Acylgruppe oder einer verzweigten oder unverzweigten Alkylgruppe substituierter oder unsubstituierter C₅-C₆-Aryl- oder Heteroarylrest ist und
- R²: ein verzweigter oder unverzweigter C₁-C₅-Alkylrest ist oder beide R²-Reste direkt miteinander oder über eine C₁-C₄-Einheit miteinander verbunden sind,
- Y: immer ein Y für Wasserstoff und das andere Y für einen -NO₂, -CN oder -CF₃-Rest oder Fluor oder Chlor steht oder beide Y-Reste unabhängig voneinander für einen NO₂-, CN- oder CF₂-Reste oder Fluor oder Chlor stehen und
- Z: für Wasserstoff oder für einen -NO₂-Rest oder verzweigten oder unverzweigten C₁-C₆-Alkylrest oder Acylrest steht,
durch Umsetzung einer 2-Hydroxyaceetalverbindung der allgemeinen Formel (II) 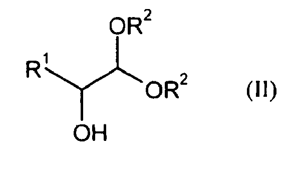 in der
- R¹ und R²: die für Formel (Ia) angegebene Bedeutung haben,
mit einem substituierten Aromaten der allgemeinen Formel (III) 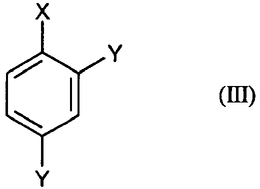 in der
- Y: die für Formel (I) angegebene Bedeutung hat und
- X: für Fluor, Chlor, Brom oder eine NO₂-Gruppe steht,
- in: Gegenwart eines Alkalimetallalkoholats oder eines festen Alkalimetallhydroxids in Kombination mit festem Alkalimetallcarbonat.

Als Alkalimetallalkoholat kann dabei Natrium- oder Kaliummethanolat-ethanolat oder tert.-Butylat, bevorzugt Kalium-tert-butylat oder festes Alkalimetallhydroxid wie Kalium- oder Natriumhydroxid in Kombination mit festem Alkalimetallcarbonat wie Natrium-, Lithium- oder Kaliumcarbonat, bevorzugt festes Kaliumcarbonat, eingesetzt werden.

Im Einzelnen wird dabei so vorgegangen, dass man in wasserfreien Lösungsmitteln wie z.B. Dimethylsulfoxid (DMSO), Dimethylacetamid (DMAc), N-Methylpyrrolidon (NMP), Tetrahydrofuran (THF) und insbesondere Dimethylformamid (DMF) ein Alkalimetallalkoholat, in einer Menge von 1,1 bis 1,2 Äquivalenten, z.B. Kalium-tert.-butylat als 10 - 50 %ige, insbesondere 20 - 30 %ige Lösung vorlegt und hierzu ein 2-Hydroxyacetal der Formel (I), z.B. 1,1-Dimethoxy-2-hexanol (1,0 Äquivalent) zudosiert. Anschließend werden 1,05 bis 1,2 Äquivalente des substituierten Aromaten der Formel (II), z.B. 4-Chlornitrobenzol als 30 %ige Lösung in einem Lösungsmittel wie DMF über einen Zeitraum von 0,25 bis 2 Stunden, insbesondere 0,5 bis 1 Stunde, zudosiert. Anschließend wird noch 1 bis 6 Stunden bei 30 bis 60°C, insbesondere 40°C, nachgerührt. Nach Herauswaschen der Salze kann dann das Lösungsmittel z.B. durch Destillation, entfernt werden. Die Ausbeuten liegen üblicherweise bei 90 - 95 % und die Reinheit bei über 90 %.

Neben DMF kann als Lösungsmittel auch ein Gemisch aus DMF mit Toluol, Chlorbenzol oder Xylol verwendet werden.. Üblicherweise liegt der Gehalt an DMF dabei 40 Gew.-% oder höher.

Als festes Alkalimetallhydroxid, kann dabei z.B. festes Kaliumhydroxid oder Natriumhydroxid in Kombination mit festem Alkalimetallcarbonat verwendet werden. Dabei kommen bevorzugt 1 bis 2 Äquivalente, und besonders bevorzugt 1.3 bis 1.6 Äquivalente des Hydroxids in fester Form zum Einsatz. Das Alkalimetallhydroxid/Alkalimetallcarbonat Verhältnis kann zwischen 1:1 und 10:1 variiert werden. Bevorzugt wird als festes Alkalimetallhydroxid festes Kaliumhydroxid und als festes Alkalimetallcarbonat festes Kaliumcarbonat eingesetzt.

Als Prozesslösungsmittel bei Einsatz von festem Kaliumhydroxid oder Natriumhydroxid in Kombination mit festem Kaliumcarbonat dient typischerweise Toluol oder Chlorbenzol, es können aber auch andere halogenierte oder nicht halogenierte aromatische oder aliphatische Kohlenwasserstoffe als Lösungsmittel in Frage kommen. Des weiteren kann die Reaktion auch in Gegenwart von 0.1 bis 1.0 Äquivalenten eines polar aprotischen Lösungsmittels durchgeführt werden. Verwendet wird dabei typischerweise Dimethylacetamid oder N-Methylpyrrolidon. Es wird normalerweise so vorgegangen, dass sämtliche Reagenzien und Lösungsmittel gemischt werden und die Reaktionsmischung auf die gewünschte Temperatur erwärmt wird. Die Reaktion wird typischerweise bei Temperaturen zwischen 40 und 100 °C durchgeführt, besonders bevorzugt bei Temperaturen zwischen 60 und 80 °C. Die Reaktionszeit beträgt typischerweise zwischen 4 und 14 Stunden. Zur Aufarbeitung wird typischerweise Wasser zur Auflösung des Salze hinzugefügt und die organische Phase noch mehrmals mit Wasser gewaschen.

Bei den nach dem erfindungsgemäßen Verfahren hergestellten 2-Phenoxyacetalen ist R¹ bevorzugt ein C₄-Alkyl oder Phenylrest. R¹ kann aber auch ein z.B. mit einem Halogen, einer -CN-, -NO₂, -CF₃, Acyl- oder Alkylgruppe, wie z.B. Ethyl- oder iso-Propyl, substituierter C₅-C₆-Aryl- oder Heteroarylrest sein. Auch unsubstituierte Arylreste wie Phenyl, oder verzweigte und unverzweigte C₁-C₁₂-Alkylreste sind möglich.

R² kann ein verzweigter oder unverzweigter C₁-C₁₂-Alkylrest wie z.B. Methylat, Ethylat, Propylat, iso-Propylat, sec- oder tert.-Butylat oder Amylrest sein. Beide R²-Reste können aber auch direkt miteinander oder über eine C₁-C₄-Einheit miteinander verbunden sein. Ein Beispiel hierfür ist z.B. ein Glykolrest.

R² ist insbesondere eine Methyl oder Ethylgruppe und die Verbindungen der allgemeinen Formel (II) somit ein 2-Aryloxy-dimethylacetal oder 2-Aryloxy-diethylacetal.

Y kann in ortho- oder para-Stellung an substituierten Aromaten stehen, d.h., ein Y in Formel (I) ist immer gleich Wasserstoff. Bevorzugt ist das andere Y eine -NO₂-Gruppe. Ansonsten kann Y noch eine CN- oder CF₃Gruppe oder Fluor oder Chlor sein. Y kann auch sowohl in ortho- als auch in para-Stellung unabhängig für jeweils eine NO₂-, CN- oder CF₃-Gruppe oder auch für Fluor oder Chlor stehen.

Z in Formel (I) und (III) kann Wasserstoff oder ein verzweigter oder unverzweigter C₁-C₆-Alkylrest, z.B. ein Methyl-, Ethyl- oder Isopropylrest sein. Bevorzugt ist Z in Formel (I) und (II) Wasserstoff.

Durch saure Hydrolyse lassen sich die erfindungsgemäß hergestellten 2-Phenoxyacetale leicht in die korrespondierenden 2-Phenoxycarbaldehyde überführen. Vorzugsweise wird die Hydrolyse dabei sofort im Anschluss an die Herstellung der 2-Phenoxyacetale durch Zugabe von Wasser und einer anorganischen (37%ige HCl) oder organische Säure wie Ameisensäure zur Reaktionslösung durchgeführt. Die Herstellung der korrespondierenden 2-Phenoxycarbaldehyde aus den erfindungsgemäß erhaltenen 2-Phenoxyacetalen ist daher ebenfalls Gegenstand dieser Erfindung.

Die auf diesem Wege erhaltenen 2-Phenoxyacetale sowie deren Carbaldehyde stellen wirtschaftlich interessante Ausgangsmaterialien für 2-Alkyl-5-nitrobenzofurane dar, die als Vorstufen von Pharmaprodukten Verwendung finden.

Daher sind die 2-Phenoxyacetale der die dann korrespondierenden 2-Phenoxycarbaldehyde ebenfalls Gegenstand der Erfindung. Bevorzugt ist als 2-Phenoxyacetal dabei 1,1-Dimethoxy-2-(4-nitrophenoxy)-hexan.

### Beispiele

### Darstellung von 1,1-Dimethoxy-2-(4-nitrophenoxy)-hexan aus 4-Chlornitrobenzol

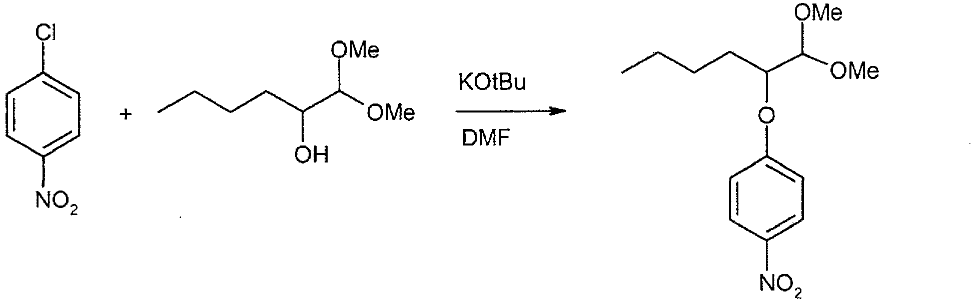

In einem 1L-Planschliffbecher wurden bei Raumtemperatur 1,1 Äquivalente Kalium-tert.-butylat als 30 %ige Lösung, in DMF vorgelegt und anschließend 1,0 Äquivalente 1,1-dimethoxy-2-hexanol innerhalb von 15 Minuten zudosiert. Der Ansatz wurde 15 Minuten gerührt und danach bei 25°C 1,1 Äquivalente 4-Chlornirobenzol als 30 %ige Lösung (nach Gewicht) in DMF innerhalb 1 Stunde zudosiert. Der Ansatz wurde anschließend zwischen 4 Stunden, bei einer Temperatur von 40°C nachgerührt. Die Reaktionsmischung wurde anschließend auf 15°C abgekühlt, mit dem gleichen Volumen Methyl-tert.-butylether extrahiert. Anschließend wurden die vereinigten organischen Phasen zweimal mit jeweils einem Drittel des Volumens der organischen Phase an Wasser gewaschen und das Lösungsmittel unter vermindertem Druck entfernt. Das gewünschte Produkt wurde als braunes Öl in 93 %iger Reinheit und 95 %iger Ausbeute erhalten.

### Darstellung von 1,1-Dimethoxy-2-(4-nitrophenoxyl-hexan aus 4-Fluornitrobenzol

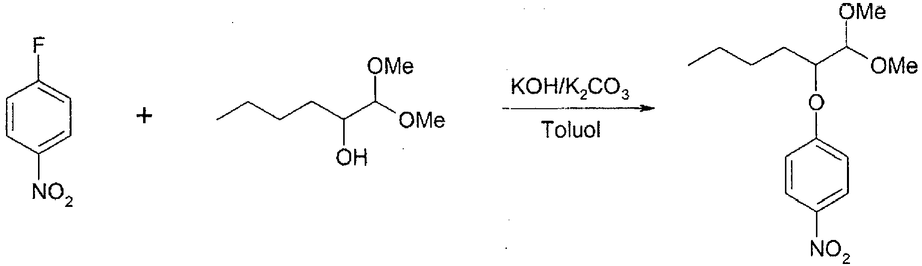

In einem 1L Planschliffbecher wurde 1 Äquivalent 1,1-Dimethoxy-2-hexanol als 30 %ige Lösung in Toluol vorgelegt und 1,05 Äquivalente 4-Fluomitrobenzol hinzugegeben. Anschließend wurden 0.33 Äquivalente N-Methylpyrrolidon, 0.12 Äquivalente festes Kaliumcarbonat, sowie 1,5 Äquivalente festes Kaliumhydroxid hinzugefügt. Die Reaktionsmischung wurde unter Rühren für 7 Stunden auf 80 °C erwärmt. Anschließend wurde auf 55 °C abgekühlt und Wasser hinzugefügt. Die wässrige Phase wurde abgetrennt und die organische Phase noch zweimal mit Wasser nachgewaschen. Nach Entfernung des Lösungsmittels wurde das Produkt in 94 %iger Reinheit und 92%iger Ausbeute erhalten.

## Patentansprüche

1. Verfahren zur Herstellung von 2-Phenoxy-acetalen der allgemeinen Formel (Ia) 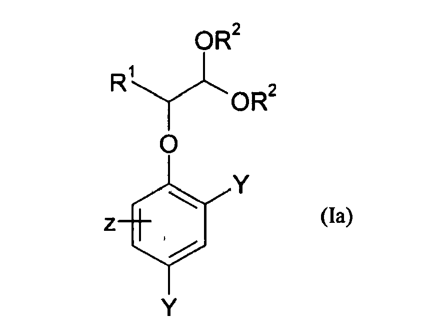 in der
R¹ ein verzweigter oder unverzweigter C₁-C₁₂-Alkylrest, ein elektronenarmer, mit Halogen, einer NO₂-, -CN, -CF₃-, Acylgruppe oder einer verzweigten oder unverzweigten Alkylgruppe substituierter oder unsubstituierter C₅-C₆-Aryl- oder Heteroarylrest ist und
R² ein verzweigter oder unverzweigter C₁-C₅-Alkylrest ist oder beide R²-Reste direkt miteinander oder über eine C₁-C₄-Einheit miteinander verbunden sind,
Y immer ein Y für Wasserstoff und das andere Y für einen -NO₂, -CN oder -CF₃-Rest oder Fluor oder Chlor steht oder beide Y-Reste jeweils unabhängig voneinander für einen NO₂-, CN- oder CF₃-Rest oder Fluor oder Chlor stehen,
Z für Wasserstoff oder für einen -NO₂-Rest oder verzweigten oder unverzweigten C₁-C₆-Alkylrest oder Acylrest steht,
durch Umsetzung einer 2-Hydroxyacetalverbindung der allgemeinen Formel (II) 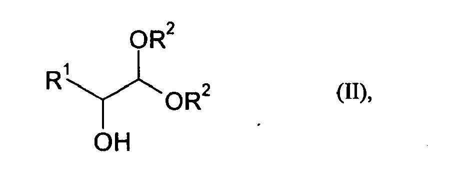 in der
R¹ und R² die für Formel (Ia) angegebene Bedeutung haben,
mit einem substituierten Aromaten der allgemeinen Formel (III) 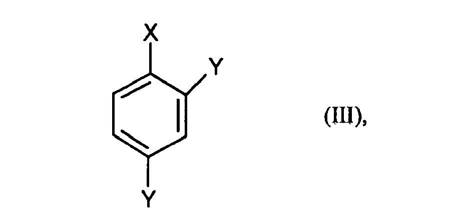 in der
Y die für Formel (I) angegebene Bedeutung hat und
X für Fluor, Chlor, Brom oder eine NO₂-Gruppe steht,
in Gegenwart eines Alkalimetallalkoholats oder eines festen Alkalimetallhydroxids in Kombination mit festem Alkalimetallcarbonat.

2. Verfahren zur Herstellung von 2-Phenoxycarbaldehyden der allgemeinen Formel (Ib), 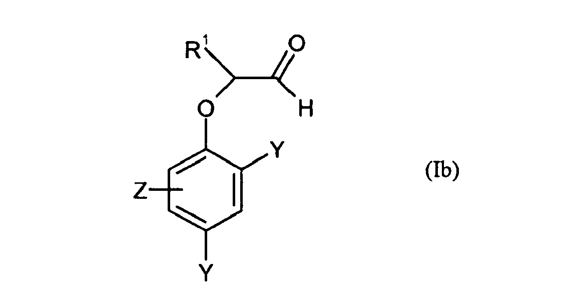 in der
R¹, Z und Y die für Formel (Ia) angegebene Bedeutung haben,
**dadurch gekennzeichnet, dass** man ein gemäß Anspruch 1 erhaltenes 2-Phenoxyacetal der Formel (Ia) einer sauren Hydrolyse zuführt.

3. Verfahren nach einem der Ansprüche 1 oder 2, **dadurch gekennzeichnet, dass** R¹ ein C₄-Alkylrest ist.

4. Verfahren nach Anspruch 1, **dadurch gekennzeichnet, dass** man die Umsetzung in wasserfreien Dimethylformamid als Lösungsmittel durchführt.

5. Verfahren nach Anspruch 1, **dadurch gekennzeichnet, dass** die 2-Hydroxyacetalverbindung der Formel (II) vor Umsetzung mit dem substituierten Aromaten der allgemeinen Formel (III) mit einem Alkoholat vorbehandelt wird.

6. 2-Phenoxyacetale der allgemeinen Formel (Ia) 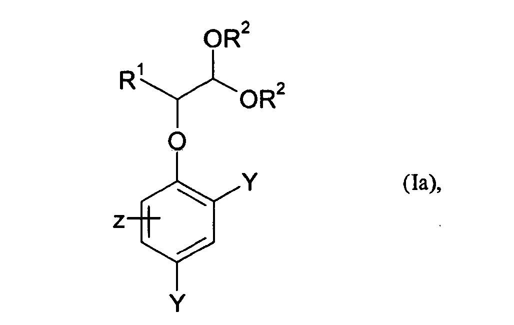 in der
R¹ ein verzweigter oder unverzweigter C₁-C₁₂-Alkylres, ein elektronenarmer, mit Halogen, einer NO₂-, -CN, -CF₃-, Acylgruppe oder einer verzweigten oder unverzweigten Alkylgruppe substituierter oder unsubstituierter C₅-C₆-Aryl- oder Heteroarylrest ist und
R² ein verzweigter oder unverzweigter C₁-C₅-Alkylrest ist,
Y immer ein Y für Wasserstoff und das andere Y für einen -NO₂, -CN oder -CF₃-Rest oder Fluor oder Chlor steht und
Z für Wasserstoff oder für einen -NO₂-Rest verzweigten oder unverzweigten C₁-C₆-Alkylrest oder Acylrest steht.

7. 2-Phenoxyacetale nach Anspruch 6, **dadurch gekennzeichnet, dass** Z Wasserstoff, ein Y eine NO₂-Gruppe, das andere Y Wasserstoff, R¹ eine C₁-C₄-Alkylgruppe und R² eine Methylgruppe ist.

8. 2-Phenoxycarbaldehyde der allgemeinen Formel (Ib) 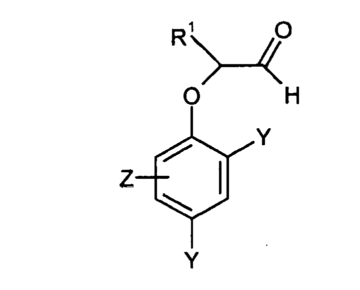 in der
R¹, Z und Y die für Formel (Ia) angegebene Bedeutung haben.

## Claims

1. Process for preparing 2-phenoxyacetals of the general formula (Ia) 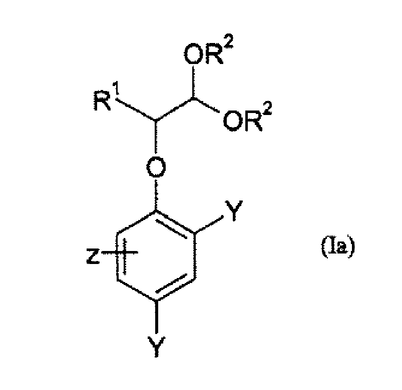 in which
R¹ is a branched or unbranched C₁-C₁₂-alkyl radical, an electron-deficient C₅-C₆-aryl or heteroaryl radical which is unsubstituted or substituted by halogen, an NO₂, -CN, -CF₃ or acyl group or a branched or unbranched alkyl group, and
R² is a branched or unbranched C₁-C₅-alkyl radical or the two R² radicals are bonded to one another directly or via a C₁-C₄ unit,
Y one Y is always hydrogen and the other Y is an -NO₂, -CN or -CF₃ radical or fluorine or chlorine, or the two Y radicals are each independently an NO₂, CN or CF₃ radical or fluorine or chlorine,
Z is hydrogen or an -NO₂ radical or branched or unbranched C₁-C₆-alkyl radical or acyl radical,
by reacting a 2-hydroxyacetal compound of the general formula (II) 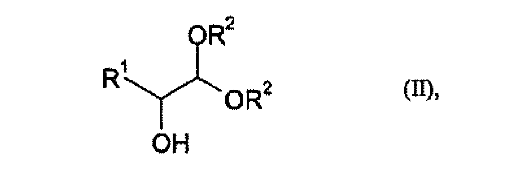 in which
R¹ and R² are each as defined for formula (Ia)
with a substituted aromatic of the general formula (III) 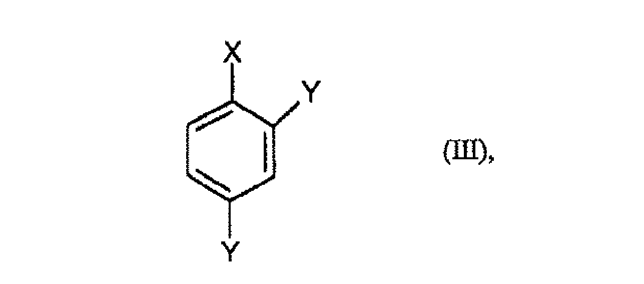 in which
Y is as defined for formula (I) and
X is fluorine, chlorine, bromine or an NO₂ group
in the presence of an alkali metal alkoxide or of a solid alkali metal hydroxide in combination with solid alkali metal carbonate.

2. Process for preparing 2-phenoxycarbaldehydes of the general formula (Ib) 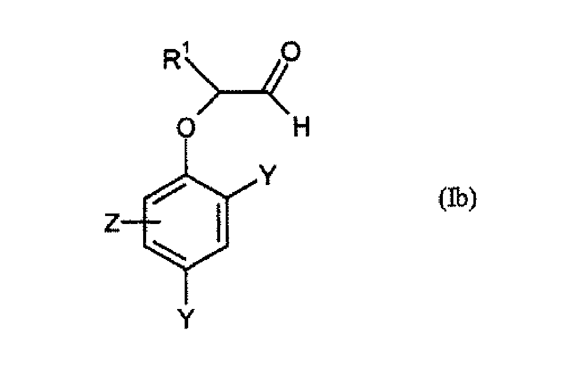 in which
R¹, Z and Y are each as defined for formula (Ia),
**characterized in that** a 2-phenoxyacetal of the formula (Ia) obtained according to Claim 1 is sent to an acidic hydrolysis.

3. Process according to either of Claims 1 and 2, **characterized in that** R¹ is a C₄-alkyl radical.

4. Process according to Claim 1, **characterized in that** the reaction is performed in anhydrous dimethylformamide as a solvent.

5. Process according to Claim 1, **characterized in that** the 2-hydroxyacetal compound of the formula (II) is pretreated with an alkoxide before reaction with the substituted aromatic of the general formula (III).

6. 2-Phenoxyacetals of the general formula (Ia) 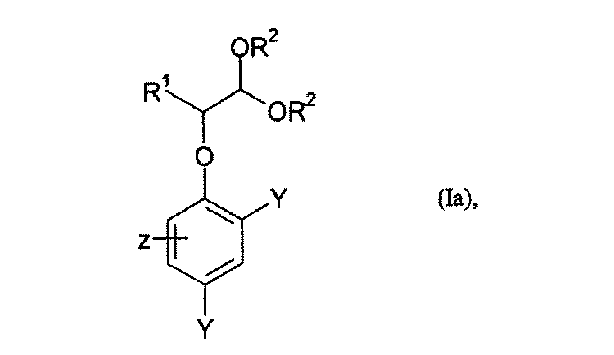 in which
R¹ is a branched or unbranched C₁-C₁₂-alkyl radical, an electron-deficient C₅-C₆-aryl or heteroaryl radical which is unsubstituted or substituted by halogen, an NO₂, -CN, -CF₃ or acyl group or a branched or unbranched alkyl group, and
R² is a branched or unbranched C₁-C₅-alkyl radical,
Y one Y is always hydrogen and the other Y is an -NO₂, -CN or -CF₃ radical or fluorine or chlorine, and
Z is hydrogen or an -NO₂ radical or branched or unbranched C₁-C₆-alkyl radical or acyl radical.

7. 2-Phenoxyacetals according to Claim 6, **characterized in that** Z is hydrogen, one Y is an NO₂ group, the other Y is hydrogen, R¹ is a C₁-C₄-alkyl group and R² is a methyl group.

8. 2-Phenoxycarbaldehydes of the general formula (Ib) 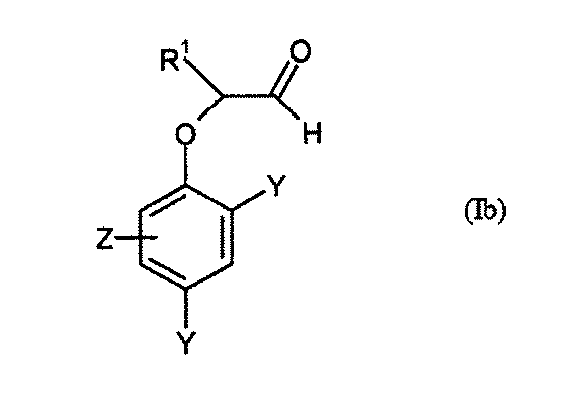 in which
R¹, Z and Y are each as defined for formula (Ia).

## Revendications

1. Procédé de fabrication de 2-phénoxyacétals de formule générale (Ia) 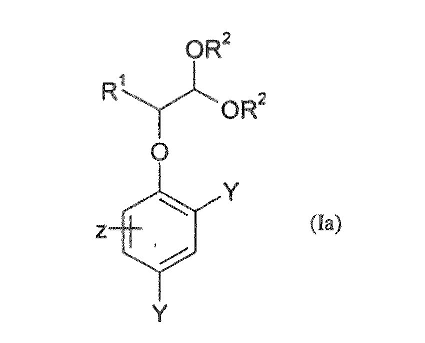 dans laquelle
R¹ est un radical alkyle en C₁-C₁₂ ramifié ou non ramifié, un radical aryle ou hétéroaryle en C₅-C₆ pauvre en électrons, non substitué ou substitué avec un halogène, un groupe NO₂, -CN, -CF₃, acyle ou un groupe alkyle ramifié ou non ramifié, et
R² est un radical alkyle en C₁-C₅ ramifié ou non ramifié ou les deux radicaux R² sont reliés l'un à l'autre directement ou par une unité en C₁-C₄,
Y un Y représente toujours l'hydrogène et l'autre Y représente un radical -NO₂, -CN ou -CF₃ ou le fluor ou le chlore, ou les deux radicaux Y représentent à chaque fois indépendamment l'un de l'autre un radical NO₂, CN ou CF₃ ou le fluor ou le chlore,
Z représente l'hydrogène ou un radical -NO₂ ou un radical alkyle en C₁-C₆ ramifié ou non ramifié ou un radical acyle,
par mise en réaction d'un composé 2-hydroxyacétal de formule générale (II) 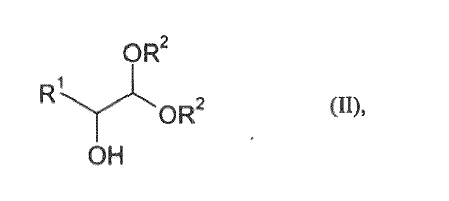 dans laquelle
R¹ et R² ont la signification indiquée pour la formule (Ia),
avec un composé aromatique substitué de formule générale (III) 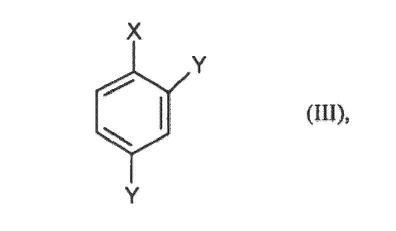 dans laquelle
Y a la signification indiquée pour la formule (I) et
X représente le fluor, le chlore, le brome ou un groupe NO₂,
en présence d'un alcoolate de métal alcalin ou d'un hydroxyde de métal alcalin solide en combinaison avec un carbonate de métal alcalin solide.

2. Procédé de fabrication de 2-phénoxycarbaldéhydes de formule générale (Ib) 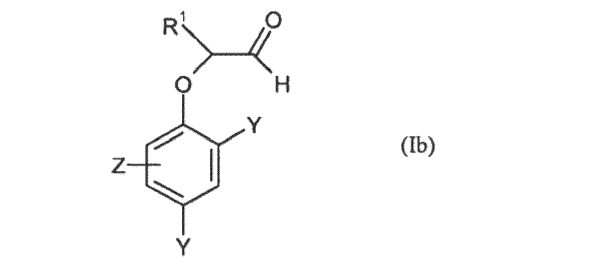 dans laquelle
R¹, Z et Y ont la signification indiquée pour la formule (Ia),
**caractérisé en ce qu'**un 2-phénoxyacétal de formule (Ia) obtenu selon la revendication 1 est introduit dans une hydrolyse acide.

3. Procédé selon l'une quelconque des revendications 1 ou 2, **caractérisé en ce que** R¹ est un radical alkyle en C₄.

4. Procédé selon la revendication 1, **caractérisé en ce que** la réaction est réalisée dans du diméthylformamide anhydre en tant que solvant.

5. Procédé selon la revendication 1, **caractérisé en ce que** le composé 2-hydroxyacétal de formule (II) est prétraité avec un alcoolate avant la réaction avec le composé aromatique substitué de formule générale (III).

6. 2-Phénoxyacétals de formule générale (Ia) 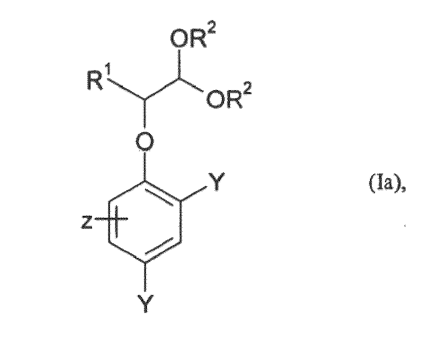 dans laquelle
R¹ est un radical alkyle en C₁-C₁₂ ramifié ou non ramifié, un radical aryle ou hétéroaryle en C₅-C₆ pauvre en électrons, non substitué ou substitué avec un halogène, un groupe NO₂, -CN, -CF₃, acyle ou un groupe alkyle ramifié ou non ramifié, et
R² est un radical alkyle en C₁-C₅ ramifié ou non ramifié,
Y un Y représente toujours l'hydrogène et l'autre Y représente un radical -NO₂, -CN ou -CF₃ ou le fluor ou le chlore, et
Z représente l'hydrogène ou un radical -NO₂ ou un radical alkyle en C₁-C₆ ramifié ou non ramifié ou un radical acyle.

7. 2-Phénoxyacétals selon la revendication 6, **caractérisés en ce que** Z est l'hydrogène, un Y est un groupe NO₂, l'autre Y est l'hydrogène, R¹ est un groupe alkyle en C₁-C₄ et R² est un groupe méthyle.

8. 2-Phénoxycarbaldéhydes de formule générale (Ib) 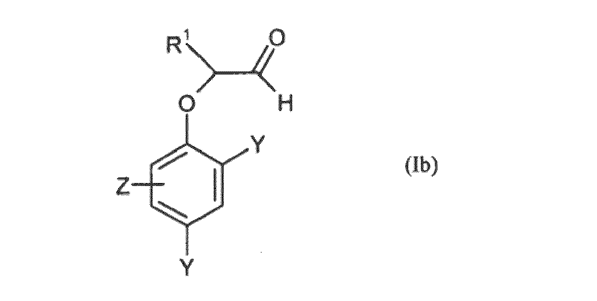 dans laquelle
R¹, Z et Y ont la signification indiquée pour la formule (Ia).
